# EUROPÄISCHE PATENTSCHRIFT

(11) **EP 1 537 841 B1**
(45) Veröffentlichungstag und Bekanntmachung des Hinweises auf die Patenterteilung: **26.03.2014**
(21) Anmeldenummer: 05005039.2
(22) Anmeldetag: 25.08.2000
(51) Int. Cl.: A61F 9/008, A61F 9/007, A61B 17/00, A61B 19/00, A61F 9/009

(54) **Vorrichtung zur Behandlung von Trübungen und/oder Verhärtungen eines ungeöffneten Auges**
Device for treating opaqueness and/or hardening of a closed eye
Dispositif de traitement de l'opacité et/ou du durcissement d'un oeil fermé

(30) Priorität: 26.08.1999 DE 19940712
(43) Veröffentlichungstag der Anmeldung: 08.06.2005
(62) Teilanmeldung aus: 00954656.5
(73) Patentinhaber: Carl Zeiss Meditec AG, 07745 Jena (DE)
(72) Erfinder: Dick, Manfred, 07926 Gefell (DE); Schröder, Eckhard, 90542 Eckental (DE)
(74) Vertreter: DTS München

(56) Entgegenhaltungen:
- WO-A-93/08677
- WO-A-94/25107
- WO-A1-98/55035
- US-A- 5 741 245

## Beschreibung

Die Erfindung betrifft eine Vorrichtung zur Behandlung von Trübungen und/ oder Verhärtungen eines ungeöffneten Auges. Insbesondere betrifft die Erfindung ein Lasersystem zur Säuberung des insbesondere alternden menschlichen Auges von Grauschleiern in der Hornhaut, der Linse oder dem Glaskörper zur Wiederherstellung der Transparenz im Auge.

In der Ophthalmologie ist es bekannt, daß es insbesondere beim alternden Auge zu Trübungen in der Linse(grauer Star) oder im Glaskörper bzw. der Cornea kommt. Die Behandlung im fortgeschrittenen Stadium beschränkt sich gegenwärtig darauf, daß man die Linse im Rahmen einer Katarakt-OP gegen eine Kunststofflinse austauscht, den Glaskörper durch Vitrektomie gegen Silikonöl austauscht oder auch die Hornhaut transplantiert. Es ist bekannt, die Operation des Kataraktes, als auch die Vitrektomie des Glaskörpers mittels Laser durchzuführen. In beiden Fällen wird der Laserstrahl im Rahmen einer Operation unmittelbar an das behandelnde Gewebe herangeführt. Als Laser hat sich besonders der Er:YAG-Laser mit der Emissionswellenlänge von 2,94 µm bewährt, dessen Strahlung sehr stark von Wasser absorbiert wird. Für den Transport der Laserstrahlung werden Kanülen mit Lichtleitern bis zum Ort der Behandlung geführt. Auch wenn mittlerweile Kanülen mit Durchmessern von ca. 1mm realisierbar sind, bleibt die Notwendigkeit des chirurgischen Eingriffs bestehen. Eine Vorrichtung zur Durchführung einer Laser-Phacoemulsifikation ist beispielsweise in DE 19718139 beschrieben.

Bekannt sind auch OP-Techniken, bei denen das Auge nicht eröffnet wird, sondern das Laserlicht über den normalen Weg des Sehvorganges in das Auge geführt wird. Hierzu zählt die Möglichkeit durch Fokussierung von fs-Laserimpulsen (300 fs, 1 µJ, 780 nm) im Inneren der Cornea eine optiche Disruption zu erzielen, welche zu Bläschenbildung führt. Durch das Aufklappen einer Lamelle kann ein intrastromales Lentikel präpariert werden, dessen Entfernung eine refraktive Korrektur bewirkt. Bekannt ist weiterhin, daß mit Hilfe von ns-Impulsen eines gütegeschalteten Nd:YAG-Lasers disruptiv das graue Nachstarhäutchen entfernt werden kann.

WO93/08677 offenbart eine Laservorrichtung nach dem Oberbegriff des angehängten Anspruchs 1.

Eine gezielte Behandlung der getrübten Areale bereits im Anfangsstadium war bisher, von medikamentösen Methoden abgesehen, nicht möglich. So sind die bekannten Lasermethoden nicht geeignet, ohne Öffnung des Auges die getrübten Areale im Auge zu beseitigen. Es ist deshalb Aufgabe der Erfindung eine Vorrichtung bereitzustellen, die es ermöglicht, getrübte Areale im Auge aufzulösen.

Eine weitere, im Alter auftretende Erscheinung ist die Presbyopie. Eine Ursache dafür besteht in der Verhärtung der Linse, die z.B. durch Einlagerung von Substanzen eintreten kann. Neben der Verwendung von Brillen, wurde neuerdings oft die Photorefraktive Keratektomie (PRK), zur Korrektur der Fehlsichtigkeit eingesetzt. Die Beseitigung der Verhärtung selbst ist bisher nicht möglich. Es ist deshalb eine weitere Aufgabe der Erfindungen eine Vorrichtung bereitzustellen, mit der die Fähigkeit zur Kontraktion der Linse wieder erhöht wird.

Aufgabe der vorliegenden Erfindung ist es daher, eine Vorrichtung bereitzustellen, mit denen Trübungen und/oder Verhärtungen eines Auges aufgelöst werden können.

Diese Aufgabe wird durch die Vorrichtung nach dem unabhängigen Anspruch gelöst. Vorteilhafte Ausgestaltungen der Erfindung sind in den Unteransprüchen angegeben.

Offenbart ist ein Verfahren zur Auflösung von Trübungen und/oder Verhärtungen eines ungeöffneten Auges gelöst, wobei die Trübungen und/oder die Verhärtungen mittels mindestens eines ultrakurzen Pulses eines Laser aufgelöst werden, ohne daß das Auge geöffnet wird. Durch den Einsatz eines ultrakurzen Pulses, der durch die transparente Augenstruktur geschickt wird, kommt es auf der Netzhaut oder anderen unbeteiligten Regionen zu keinen thermischen oder athermischen Schäden. In der Arbeitsebene (z.B. der Linse, dem Glaskörper oder in der Cornea) herrscht eine derartige Energiedichte, daß zwar im volltransparenten Medium des Auges nichts passiert, aber an heterogenen punktuellen Eintrübungen durch lokale Absorption Disruptionen induziert werden, die zur Auflösung dieser Verunreinigungen führen. Diese Disruptionen führen zur Evaporation dieser Verunreinigungen. Die hierbei eventuell enstehenden Gasbläschen (Kaviolen) werden in wenigen Stunden aufgefüllt und sind damit verschwunden. Die aufgelösten Verunreinigungen werden durch Resorption und/oder Versprengung reduziert oder verschwinden ganz.

Als ultrakurze Pulse werden bevorzugt Pulse eingesetzt, die im ps-Bereich liegen, besonders bevorzugt Pulse, die im fs-Bereich liegen. Bevorzugt werden Pulse von 10 ps bis 10 fs, besonders bevorzugt von 300 fs eingesetzt.

Der besondere Vorteil des offenbarten Verfahrens besteht darin, daß die Trübungen und/oder Verhärtungen am Auge entfernt bzw. reduziert werden können, ohne daß es erforderlich wäre, das Auge zu öffnen. Auf diese Weise werden die mit einer Operation verbundenen Risiken vermieden. Darüber hinaus kann mit dem Verfahren durch entsprechende Wahl der Energie des ultrakurzen Pulses eine schonendere und in kleinen Stufen erfolgende Behandlung vorgenommen werden.

Bevorzugt werden die ultrakurzen Pulse nachverstärkt, insbesondere bevorzugt mit der Chirped Pulse Amplification Methode (CPA-Methode).

Bei einem bevorzugten offenbarten Verfahren werden die Trübungen und/oder die Verhärtungen mittels mindestens eines Impulszuges von einer Dauer von weniger als 5s, bevorzugt weniger als 3s, besonders bevorzugt von weniger als 0,1s der ultrakurzen Pulse aufgelöst. Ganz besonders bevorzugt sind Pulslängen im Bereich von 10 ps bis 10 fs und insbesondere bevorzugt von etwa 300 fs vorgesehen. Durch die Wahl eines Impulszuges kann über die Festlegung der Dauer der Energieeintrag in dem zu behandelnden Areal vorgestimmt werden. Durch die Wahl extrem kurzer Impulszüge ist es darüberhinaus möglich, Effizienzverluste zu vermeiden, die beispielsweise durch eine Bewegung des Auges während der Behandlung eintreten könnte. Insbesondere bevorzugt weisen die Impulse eine Dauer von weniger als 10 ps auf. Es ist ebenfalls denkbar, den Impulszug im Dauerbetrieb so lange anzuwenden, bis die gewünschte Wirkung erzielt wurde. Ganz besonders bevorzugt können auch Einzelimpulse und sehr kurze Impulszüge eingesetzt werden, um durch eine iterative Nachkontrolle des Behandlungserfolges eine besonders schonende Behandlung zu erreichen.

Bei einem weiteren bevorzugten Verfahren werden Impulszüge mit einer Folgefrequenz ausgesendet, insbesondere mit einer Folgefrequenz im kHz-Bereich. Hierbei werden die Impulszüge selbst noch einmal mit einer Folgefrequenz überlagert. Auf diese Weise kann trotz der Wahl eines längeren Impulszuges oder gar eines Dauerbetriebes der Energieeintrag in das zu behandelnde Areal nochmals zeitlich variiert werden. Dadurch ist eine nochmals schonendere Behandlung unter Vermeidung jeglicher thermischer oder athermischer Schäden am Auge in Bereichen, die nicht behandelt werden sollen, möglich.

Bei einem weiteren bevorzugten Verfahren wird eine Laserstrahlung einer Wellenlängenverteilung gewählt, die für die Trübungen und/oder Verhärtungen eine höhere Absorption und/oder eine niedrigere Reflexion aufweist, als für die übrigen Bestandteile des Auges. Dadurch ist es möglich, die Energiedichte derart einzustellen, daß nur an Orten lokaler Absorption die notwendige Dichte zum Zünden eines optischen Durchbruchs erreicht wird. Diese selektive Einstellung wird durch die erhöhte Absorption der Eintrübungen und/oder der Verhärtungen bei den gewählten Wellenlängen erreicht. Besonders bevorzugt wird ein Laser ausgewählt, für dessen Wellenlänge das Auge ein hohes Transmissionsvermögen besitzt. Bevorzugt beträgt die Wellenlänge 350 bis 1300 nm. Im ganz bevorzugter Weise wird ein Laser ausgewählt, für dessen Strahlung die sensiblen Bereiche wie die Netzhaut oder die Makula eine etwas geringere Empfindlichkeit auweisen. Dies kann bevorzugt durch ein geringeres Absoptionsvermögen dieser Bereiche im Auge für die gewählte Strahlung erfolgen. Weiterhin bevorzugt kann dies durch ein höheres Reflexionsvermögen der nicht zu behandelnden Bereiche des Auges geschehen. Die Strahlung kann so unabhängig von der durch Fokussierung erzeugbaren Energiedichte aufgrund des Absorptions- und Reflexionsverhaltens schon keinen Schaden in den nicht zu behandelnden Bereichen des Auges anrichten.

Bei einem weiteren bevorzugten Verfahren werden ultrakurzen Pulse so ausgerichtet, daß innerhalb der Trübungen und/oder Verhärtungen Energiedichten auftreten, die die Trübungen und/oder Verhärtungen auflösen und gleichzeitig in sensiblen Bereich des Auges keine Zerstörung des Gewebes eintritt. Dies kann neben der Wahl der Wellenlänge durch eine Fokussierung des Strahles und eine entsprechende Strahlführung der Pulse erfolgen. So können durch eine Formung der Strahlgeometrie des Pulses im Bereich des zu behandelnden Gewebes Energiedichten eingekoppelt werden, die zu einer Disruption (und damit zur Auflösung) des krankhaften (weniger transparenten) Gewebes führen. Gleichzeitig kann der Strahl so geformt werden, daß im Bereich sensibler Bereiche, wie der Netzhaut und insbesondere der Makula, Energiedichten auftreten, die nicht zur Zerstörung dieses Gewebes führen.

Dies kann bevorzugt durch die Strahlführung erreicht werden, indem nach dem Durchgang des Strahls durch den zu behandelnden Zielbereich der Strahl so aufgeweitet wird, daß die Energiedichten im sensiblen Bereich so gering sind, daß es nicht zur Schädigung des Bereichs kommen kann. Bei einem weiteren bevorzugten Verfahren wird die komplette oder ein vorbestimmt großer Bereich der Augenlinse mit einem konvergenten Strahlbündel und einer Energiedichte im Linsenbereich unter der des optischen Durchbruchs bestrahlt. Der Fokus liegt hierbei im Glaskörper. Die Energie wird andererseits so gewählt, daß bei Transparenz der Linse im Fokus im Glaskörper ein optischer Durchbruch entsteht. Da beim optischen Durchbruch im Fokus sämtliche Energie verzehrt wird, kann bezüglich der Makula eine hohe Behandlungssicherheit hergestellt werden. Etwaige Blasenbildungen im Glaskörper relaxieren kurzfristig.

Bei einem weiteren bevorzugten Verfahren erfolgt die Ausrichtung der ultrakurzen Pulse durch eine Ablenkeinrichtung und/oder eine Fokussieroptik und/oder ein Kontaktglas. Hierdurch können die ultrakurzen Pulse und der hierdurch beschriebene Strahl nicht nur genau auf das zu behandelnde Areal ausgerichtet werden, sondern es kann darüberhinaus auch die im Zielgebiet gewünschte Energiedichte vorgewählt werden. Durch den Rückgriff auf bekannte Vorrichtungen kann das Verfahren kostengünstig umgesetzt werden.

Bei einem weiteren bevorzugten Verfahren werden vor der eigentlichen Behandlung durch eine Messung von reflektierter Strahlung niedriger Energie Informationen über die Trübungen und/oder Verhärtungen gewonnen und diese gewonnenen Informationen fließen in die Wahl der Ausrichtungen der Energie der einzusetzenden Pulse ein. Zum Schutz der sensiblen Bereiche liegt es nämlich insbesondere auch im Rahmen der Erfindung, vor der eigentlichen Behandlungsstrahlung mit wesentlich geringerem, für das Auge unschädlichen Insentitäten einzustrahlen und aus der Strahlung, die zum Beispiel an den Eintrübungen reflektiert wird, Rückschlüsse zur Ausrichtung des Lasers, sowie zur erforderlichen Strahlungsdosis in der jeweiligen Strahlungsrichtung abzuleiten. Da sich die Energie der Strahlung bei der Disruption von Eintrübungen weitgehend aufbraucht, ist eine optimale Anpassung der Strahlgeometrie auf die zu behandelnden Eintrübungen auch vorteilhaft bei der schonenden Behandlung der sensiblen Bereiche. Durch die so gewonnenen Informationen lassen sich die erkannten Areale individuell und zielgerichtet behandeln. Insbesondere ist diese Informationsgewinnung auch zwischen den einzelnen Behandlungsschritten möglich, um festzustellen, inwieweit die Behandlung bereits Erfolg gezeigt hat. So ist es beispielsweise möglich einem ultrakurzen Puls oder einem Impulszug ein Signal mit geringerer Engerie hinterherzuschicken, um hieraus Informationen über die durch den ultrakurzen Puls bzw. Impulszug bewirkte Änderung im behandelnden Areal zu gewinnen.

Bei einem weiteren Verfahren zur Behandlung der Presbyopie eines Auges werden in der Linse des Auges Bläschen erzeugt und diese Bläschen durch Flüssigkeit aufgefüllt, ohne daß das Auge geöffnet werden müßte. Durch diese Bläschenbildung innerhalb der Linse wird das Linsenmaterial gelockert. Die so gebildeten Bläschen werden automatisch durch Flüssigkeit wieder aufgefüllt. Durch diese mit Flüssigkeit gefüllten Bläschen wird eine Linse geschaffen, die ein höhere Flexibilität aufweist, als die ursprüngliche Linse. Dadurch aber wird die Akkomodation der Linse erhöht.

So sind insbesondere Einlagerungen oder Verhärtungen, die zu einer Verminderung der Kontraktionsfähigkeit der Linse führen, mit dem Verfahren behandelbar.

Besonders bevorzugt werden die Bläschen im Randbereich der Linse als Bläschenfelder erzeugt. Dieses Setzen von Bläschen im Randbereich bzw. in der Randzone der Linse führt nach Auffüllung mit Flüssigkeit zu einer Aufweichung der Linse. Dies führt zu einer höheren Flexibilität und damit zu einer höheren Akkommodation der Linse. Durch eine symmetrische Anordnung der Bläschenfelder kann die Akkommodationsmöglichkeit der Linse symmetrisch erhalten bleiben. Liegt eine nur teilweise Verhärtung der Linse vor, so kann durch gezielte Bläschenbildung die Flexibilität der Linse in einem speziellen Bereich erhöht werden. Hierdurch kann die Gesamtsymmetrie der Linse bei der Akkommodation verbessert werden.

Die Aufgabe der vorliegenden Erfindung wird weiterhin gelöst durch eine Vorrichtung zur Behandlung von Trübungen und/oder Verhärtungen eines ungeöffneten Auges umfassend eine kohärente Lichtquelle, wobei die kohärente Lichtquelle eine Einrichtung zur Erzeugung von ultrakurzen Pulsen aufweist. Durch diese ultrakurzen Pulse können die vorstehenden Vorteile bei dem Verfahren umgesetzt werden.

Die kohärente Lichtquelle umfasst einen Laser. Dieser Laser wird so gewählt, daß er Pulse im ps-Bereich, bevorzugt im fs-Bereich abstrahlen kann.

Besonders bevorzugt weist die kohärente Lichtquelle eine Einrichtung zur Erzeugung mindestens eines Impulszuges vor. Dieser Impulszug hat bevorzugt eine Dauer von weniger als 5s, besonders bevorzugt weniger als 2s und insbesondere bevorzugt von weniger als 0,1s. Insbesondere bevorzugt sind Impulslängen im Bereich von 10 ps bis 10 fs vorgesehen und ganz besonders bevorzugt Impulslängen von etwa 300 fs. Bevorzugt kann die erfindungsgemäße Vorrichtung auch im Dauerbetrieb Impulszüge bereitstellen oder Einzelimpulse aussenden.

Besonders bevorzugt umfaßt die kohärente Lichtquelle eine Einrichtung zur Erzeugung von Impulszügen mit einer Folgefrequenz, insbesondere bevorzugt im kHz-Bereich. Damit ist es möglich, die im erfindungsgemäßen Verfahren beschriebene Überlagerung der einzelnen Impulszüge durch die Folgefrequenz zu erzeugen, was die schonende Einbringung der Energie in das zu behandelnde Areal erhöht.

Besonders bevorzugt weist die kohärente Lichtquelle eine Einrichtung zur Erzeugung einer Laserstrahlung mit einer Frequenzverteilung auf, die für die Trübungen und/oder Verhärtungen eine höhere Absorption und/oder eine niedrigere Reflexion aufweist, als für die übrigen Bestandteile des Auges. Insbesondere bevorzugt wird dafür ein durchstimmbarer Laser benutz, der im Bereich 350 nm bis 1300 nm abstrahlen kann. Besonders bevorzugt wird ein Laser vorgesehen, der im Bereich von 780 nm strahlen kann wie beispielsweise ein Ti-Saphir-Laser oder weiterhin bevorzugt im Bereich 1060 nm, wie beispielsweise ein Nd:Glas-Laser. Durch einen solchen Laser können die Vorteile des erfindungsgemäßen Verfahrens erzielt werden.

Bei der vorliegenden Erfindung ist eine Einrichtung zur Ausrichtung der ultrakurzen Pulse vorgesehen, umfassend eine Ablenkeinrichtung und/oder eine Fokussieroptik und/oder ein Kontaktglas. Die optischen Mittel zum Einkoppeln der Strahlung bestehen bevorzugt aus einer durchstimmbaren Fokussieroptik, Umlenkspiegeln eines Mikromanipulators, Kontaktgläser, speziellen Spiegelkontaktgläser und OP-Mikroskopen bzw. Spaltlampen. Mittels dieser Elemente ist es möglich, den Strahl innerhalb des Auges so ein- und auszurichten, daß der Energieeintrag in den zu behandelnden Arealen sehr genau vorbestimmbar sind, ohne das außerhalb dieser zu behandelnden Areale eine für das dort vorhandene Gewebe schädliche Energiedichte auftreten könnte. In einem weiteren bevorzugten Ausführungsbeispiel der vorliegenden Erfindung ist eine Steuereinrichtung vorgesehen, mit der die Einrichtung zur Ausrichtung der ultrakurzen Pulse steuerbar ist, insbesondere bevorzugt in Abhängigkeit von Informationen über die Trübungen und/oder Verhärtungen. Durch diese Steuereinrichtung können die Informationen, die über die zu behandelnden Areale ermittelt wurden, so aufbereitet werden, daß die Pulsdauer, Abfolge und einzubringende Energiedichte bestimmt werden kann und die Steuereinrichtung anhand der ermittelten Parameter die Einrichtung zur Ausrichtung der ultrakurzen Pulse dadurch ein- und ausrichten kann, daß die einzelnen Elemente des optischen Systems durch Steuereinrichtung so eingestellt werden, daß das gewünschte Areal mit dem vorbestimmten Energieeintrag behandelt werden kann.

Offenbart ist die Verwendung einer erfindungsgemäßen Vorrichtung bzw. eines offenbarten Verfahrens zur Behandlung von Hazebildung in der Hornhaut nach Eximerlaser-Behandlungen, bei Linsenkerneintrübungen bei beginnenden Katarakt und/oder bei Glaskörperverunreinigungen im Gesichtsfeld.

Ausführungsbeispiele der Erfindung und vorteilhafte Ausgestaltungen sollen im Folgenden anhand von Zeichnungen näher erläutert werden. Dabei zeigen:
Fig. 1 Ein Ausführungsbeispiel der erfindungsgemäßen Vorrichtung zur Behandlung einer Trübung im Gesichtsfeld des Glaskörpers;
Fig. 2 Ein Ausführungsbeispiel der vorliegenden Erfindung zur Behandlung der Presbyopie;
Fig. 3 Ein weiteres Ausführungsbeispiel der vorliegenden Erfindung zur Behandlung der Augenlinse;
Fig. 4 Ein weiteres Ausführungsbeispiel der vorliegenden Erfindung zur Behandlung eines speziellen Bereichs der Augenlinse und
Fig. 5 ein Diagramm eines Impulszuges mit Darstellung der Zeitachse und der Amplitude.

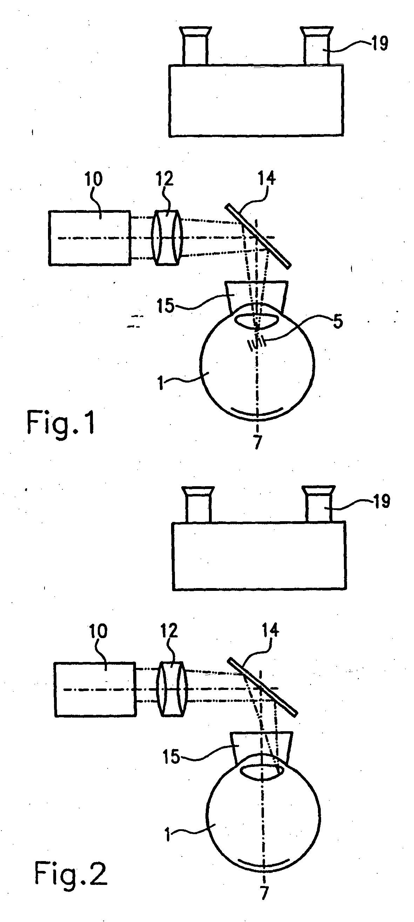

Figur 1 zeigt ein erstes Ausführungsbeispiel zur Behandlung von einer Trübung im Glaskörper im Gesichtsfeld direkt hinter der Linse. Einem Laser 10, hier einem modensynchronisierten Laser, ist eine Fokussieroptik 12 nachgeschaltet. Hinter der Fokussieroptik ist ein Umlenkspiegel mit Mikromanipulator 14 angeordnet. Auf dem zu behandelnden Auge 1 ist ein Kontaktglas 15 aufgebracht. Hinter der Augenlinse befindet sich ein getrübtes Areal 5. Zur Beobachtung dient ein OP-Mikroskop mit Spaltlampe 19.

Mit dem modensynchronisierten Lasersystem werden ultrakurze Laserimpulse vorzugsweise 10 ps bis 10 fs erzeugt, welche mit der Chirped Pulse Amplification Methode nachverstärkt werden, um im kHz-Bereich Pulsenergien größer 1 mJ verfügbar zu haben. Bei der Wellenlänge von 780 nm (Ti-Saphir) oder 1060 nm (Nd:Glas) besitzen die transparenten Areale der zu behandelnden Cornea, Linse oder des Glaskörpers eine geringe Absorption, die bei Bestrahlung mit genügend niedrigen Energiedichten des ultrakurzen Pulses nicht geschädigt werden. Hinter dem Laser 10 ist eine Fokussiereinrichtung 12 angeordnet, durch die der Strahl ausgerichtet und fokussiert wird. Der Strahl wird über den Umlenkspiegel mit Mikromanipulator 14 durch das Kontaktglas 15 auf das getrübte Areal 5 fokussiert.

Im Betrieb strahlt der Laser Impulszüge 25 ultrakurzer Pulse 20 aus. Diese werden nur von den krankhaften getrübten Arealen absorbiert wodurch eine selektive Behandlung ermöglicht wird. Die ultrakurzen Pulse führen dabei zu einem lokal begrenzten, disruptiven Zerkleinerungsprozeß des getrübten Gewebes ohne schädliche thermische Nebenwirkungen. Der lokale, selektive und athermische Zerkleinerungsprozeß führt nach Auffüllung der induzierten Bläschen zur Wiederherstellung der Transparenz in diesem Areal. Die im Glaskörper eventuell entstehenden Kavitationen werden innerhalb kurzer Zeit wieder vom Körper mit Flüssigkeit aufgefüllt. Hierdurch wird der Bereich 5 nach der Behandlung wieder transparent.

Bei entsprechender Energievorwahl können auch getrübte Areale in der Augenlinse mit dieser Anordnung behandelt werden. Die Energie wird dabei so gewählt, daß die transparenten Bestandteile der Augenlinse keine Absorption der gewählten Wellenlänge zulassen. Die getrübten Areale in der Augenlinse jedoch absorbieren die Strahlung und so führen die ultrakurzen Pulse zu einem lokal begrenzten, disruptiven Zerkleinerungsprozeß des getrübten Gewebes auch in der Augenlinse ohne schädliche thermische Nebenwirkungen. Die Energie, die durch die getrübten Areale nicht absorbiert wurden, werden im Fokus im Glaskörper durch Disportion aufgebraucht und können so die Netzhaut nicht schädigen. Die im Fokus im Glaskörper entstehenden Kavitationen werden zeitnah mit Flüssigkeit des Körpers wieder aufgefüllt und sind damit wieder transparent.

In Figur 2 ist ein Ausführungsbeispiel der vorliegenden Erfindung zur Behandlung der Presbyopie dargestellt. Die Vorrichtung entspricht im wesentlichen der in Figur 1. Jedoch erfolgt die Strahlablenkung der Impulsfolge durch den Umlenkspiegel mit Mikromanipulator 14 derart, daß der Fokus im Randbereich der Linse zu liegen kommt. Auf diese Weise können erfindungsgemäß die Bläschen, bevorzugt im Randbereich der Linse, erzeugt werden, die nach Auffüllung durch bevorzugt körpereigene Flüssigkeit eine höhere Flexibilität und damit Akkommodationsfähigkeit aufweisen. Auf diese Weise können ganze Bläschenfelder gesetzt werden, die zu einer regionalen Erweichung der Linse und damit zu einer entsprechenden Erhöhung der Flexibilität führen.

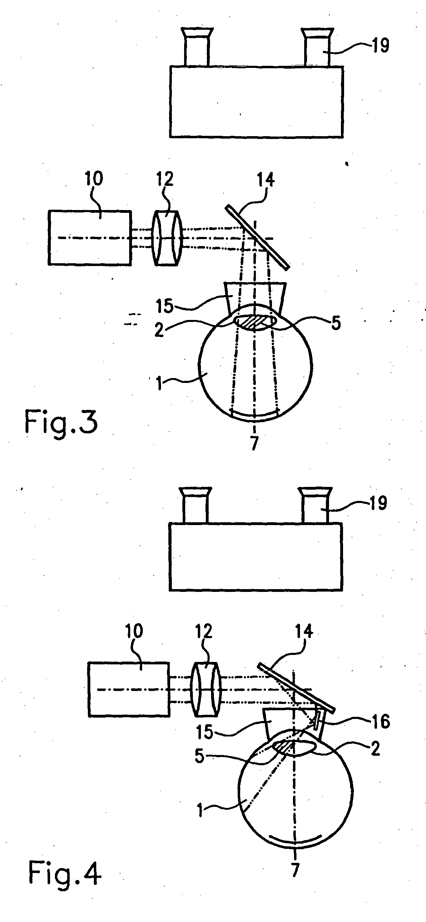

In Figur 3 ist ein weiteres Ausführungsbeispiel zur Behandlung der Augenlinse dargestellt. Auch dieses Ausführungsbeispiel entspricht im wesentlichen Aufbau dem des in Figur 1 dargestellten Ausführungsbeispieles. Jedoch wird durch das hier verwendete optische System 12 der Strahl derart aufgeweitet, daß er im Bereich der Augenlinse 2 so einstellbar ist, daß hier ein Energieeintrag erfolgt, der zu einer Zerstörung der getrübten Areale 5 in der Linse 2 führt, während im weiteren Verlauf der Strahl so aufgeweitet wird, daß die Energie im Bereich der Makula 7 so gering ist, daß hier kein Schaden an dem Gewebe angerichtet werden kann.

Bei der Behandlung wird durch spezielle divergente Strahlführung sowie geeigneter Einstrahlung sowie möglicher automatisierter Scanverfahren die Strahlung so geführt, daß weder die Netzhaut noch andere als die krankhaften Stellen geschädigt werden können.

In Figur 4 ist ein weiteres Ausführungsbeispiel zur Behandlung eines speziellen Bereichs der Augenlinse 2 dargestellt. Hierbei ist in dem Kontaktglas 15 ein Spiegel 16 vorgesehen, mit dessen Hilfe der Impulszug auf einen speziellen Bereich der Augenlinse ausgerichtet werden kann. Der Strahl trifft auf den Umlenkspiegel mit Mikromanipulator 14, der den Strahl durch das Kontaktglas 15 auf den Spiegel 16 im Kontaktglas 15 einrichtet, durch den der Strahl in den Bereich der Augenlinse 2 ausgerichtet wird, in dem das getrübte Areal 5 vorliegen.

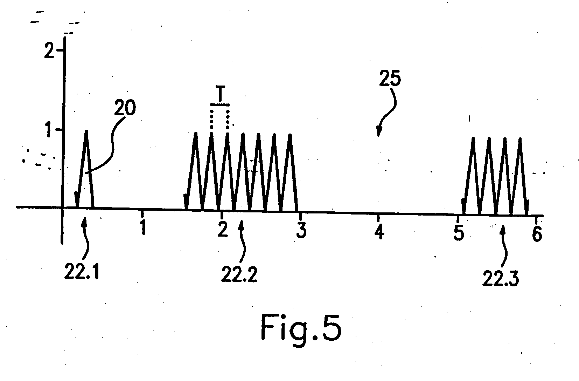

In Figur 5 ist ein Diagramm eines Impulszuges 25 mit Darstellung der Zeitachse und Amplitude dargestellt. Die einzelnen ultrakurzen Pulse 20 weisen eine Breite von einigen fento-Sekunden auf. Der Impulszug 25 wird von drei Impulspaketen 22 unterschiedlicher Längen 22.1, 22.2 und 22.3 gebildet und von einer Frequenzfolge mit der Periode T überlagert. Auf diese Weise kann der Energieeintrag durch die ultrakurzen Pulse nochmals variiert werden. Während auf der x-Achse die Zeit t dargestellt ist, ist auf der y-Achse die Amplitude A angegeben. An Stelle einer Frequenzfolge im kHz-Bereich kann auch an eine lineare oder quasi-lineare Anstiegshüllkurve bzw. absteigende Hüllkurve gedacht werden. Das erste Impulspaket 22.1 besteht aus einem Einzelpuls 20. Der Impulszug 22.2 besteht aus mehreren Einzelpulse, die selbst um die Zeit T voneinander beabstandet sind. T liegt üblicherweise im ms-Bereich, während die Breite der Einzelpulse 20 im fs-Bereich liegt.
Zusammen mit dem Impulspaket 22.3 bilden die Impulspakete den Impulszug 25.

Erfindungsgemäß wurde eine Vorrichtung zur Behandlung von Trübungen und/oder Verhärtungen eines ungeöffneten Auges vorgestellt. Ein besonderer Vorteil der erfindungsgemäßen Lösung besteht darin, daß Behandlungen im Innern des Auges möglich sind, ohne daß ein chirurgisches Instrument in das Auge eingeführt werden muß.

Bezugszeichenliste,
- 1.: Auge
- 2.: Linse
- 3.: Glaskörper
- 4.: Cornea
- 5.: Trübungen
- 7.: Makula
- 10.: Laser
- 12.: optisches System (Fokussieroptik)
- 14.: Umlenkspiegel mit Mikromanipulator
- 15.: Kontaktglas
- 16.: Spiegel im Kontaktglas
- 19.: OP-Mikroskop mit Spaltlampe
- 20.: Ultrakurzer Puls
- 22.: Impulspaket
- 25.: Impulszug

## Patentansprüche

1. Vorrichtung zur Behandlung der Presbyopie eines Auges, mit einer flexiblen Linse (2) aufweisend:
einen Laser (10) zur Erzeugung von ultrakurzen Pulsen (20), mit einer Wellenlänge im Bereich von 350 nm bis 1300 nm und
eine Einrichtung zur Ausrichtung der ultrakurzen Pulse, umfassend eine Ablenkeinrichtung (14) und/oder eine Fokussieroptik (12) und/oder ein Kontaktglas (15)
**dadurch gekennzeichnet, dass**
eine Steuereinrichtung vorgesehen ist, die die Einrichtung zur Ausrichtung der ultrakurzen Pulse so steuert, dass in der Linse (2) Bläschenfelder erzeugt werden, wobei die Bläschen automatisch durch Flüssigkeit wieder aufgefüllt werden, die zu einer lokalen Erweichung der Linse (2) und damit zu einer Erhöhung der Flexibilität der Linse führen.

2. Vorrichtung nach Anspruch 1,
**dadurch gekennzeichnet, dass** die Steuereinrichtung die Einrichtung zur Ausrichtung der ultrakurzen Pulse so steuert, dass durch gezielte Bläschenbildung die Flexibilität der Linse in einem speziellen Bereich erhöht wird.

3. Vorrichtung nach einem der vorhergehenden Ansprüche,
**dadurch gekennzeichnet, dass** die Steuereinrichtung die Einrichtung zur Ausrichtung der ultrakurzen Pulse so steuert, dass eine symmetrische Anordnung der Bläschenfelder erfolgt.

4. Vorrichtung nach einem der vorhergehenden Ansprüche,
**dadurch gekennzeichnet, dass** der Laser eingerichtet ist, mindestens einen Impulszug (25) von einer Dauer von weniger als 5 Sekunden, bevorzugt weniger als 2 Sekunden, besonders bevorzugt von weniger als 0,1 Sekunden der ultrakurzen Pulse (20) aufweist.

5. Vorrichtung nach einem der vorhergehenden Ansprüche,
**dadurch gekennzeichnet, dass** der Laser eingerichtet ist, Impulszüge (25) mit einer Folgefrequenz, insbesondere mit einer Folgefrequenz im kHz-Bereich zu erzeugen.

6. Vorrichtung nach einem der vorhergehenden Ansprüche,
**dadurch gekennzeichnet, dass** der Laser (10) eine Einrichtung zur Erzeugung einer Laserstrahlung mit einer Frequenzverteilung im Bereich von 780 nm bis 1060 nm aufweist.

## Claims

1. Device for treating presbyopia of an eye, with a flexible lens (2) having:
a laser (10) for generating ultra-short pulses (20), with a wavelength in the range of from 350 nm to 1300 nm and
equipment for aligning the ultra-short pulses, comprising a deflector (14) and/or focussing optics (12) and/or a contact glass (15)
**characterized in that**
a controller is provided, which controls the equipment for aligning the ultra-short pulses such that blistered areas are produced in the lens (2), wherein the blisters are automatically refilled with fluid, which leads to a local softening of the lens (2) and thus to an increase in the flexibility of the lens.

2. Device according to claim 1,
**characterized in that** the controller controls the equipment for aligning the ultra-short pulses such that, by targeted blister formation, the flexibility of the lens is increased in a particular area.

3. Device according to one of the previous claims, **characterized in that** the controller controls the equipment for aligning the ultra-short pulses such that the blistered areas are arranged symmetrically.

4. Device according to one of the previous claims, **characterized in that** the laser is set up, having at least one pulse train (25) of a duration of less than 5 seconds, preferably less than 2 seconds, particularly preferably less than 0.1 seconds of the ultra-short pulses (20).

5. Device according to one of the previous claims, **characterized in that** the laser is set up to generate pulse trains (25) with a repetition frequency, in particular with a repetition frequency in the kHz range.

6. Device according to one of the previous claims, **characterized in that** the laser (10) has equipment for generating a laser radiation with a frequency distribution in the range of from 780 nm to 1060 nm.

## Revendications

1. Dispositif de traitement de la presbytie d'un oeil, avec une lentille flexible (2) comportant :
un laser (10) servant à produire des impulsions ultracourtes (20), avec une longueur d'onde située dans la plage de 350 nm à 1300 nm ; et
un dispositif d'orientation des impulsions ultracourtes, comprenant un dispositif de déviation (14) et/ou un système optique de focalisation (12) et/ou un verre de contact (15) ;
**caractérisé en ce que** :
un dispositif de commande est prévu commandant le dispositif d'orientation des impulsions ultracourtes de façon à produire dans la lentille (2) des champs de petites bulles, les petites bulles étant automatiquement reremplies de fluide conduisant à un ramollissement local de la lentille (2) et ainsi à une augmentation de la flexibilité de la lentille.

2. Dispositif selon la revendication 1, **caractérisé en ce que** le dispositif de commande commande de telle sorte le dispositif d'orientation des impulsions ultracourtes que la formation ciblée de petites bulles augmente la flexibilité de la lentille dans une zone spécifique.

3. Dispositif selon l'une quelconque des revendications précédentes, **caractérisé en ce que** le dispositif de commande commande le dispositif d'orientation des impulsions ultracourtes de façon à réaliser un agencement symétrique des champs de petites bulles.

4. Dispositif selon l'une quelconque des revendications précédentes, **caractérisé en ce que** le laser est conçu pour présenter au moins un train d'impulsions (25) d'une durée inférieure à 5 secondes, de façon préférée inférieure à 2 secondes, de façon particulièrement préférée inférieure à 0,1 seconde des impulsions ultracourtes (20).

5. Dispositif selon l'une quelconque des revendications précédentes, **caractérisé en ce que** le laser est conçu pour produire des trains d'impulsions (25) présentant une certaine fréquence de répétition, notamment une fréquence de répétition dans la plage du kHz.

6. Dispositif selon l'une quelconque des revendications précédentes, **caractérisé en ce que** le laser (10) comporte un dispositif permettant de produire un rayonnement laser avec une répartition de fréquence comprise dans la plage de 780 nm à 1060 nm.
